# EUROPEAN PATENT APPLICATION

(11) **EP 4 295 843 A1**
(43) Date of publication of application: **27.12.2023**
(21) Application number: 23177772.3
(22) Date of filing: 06.06.2023
(51) Int. Cl.: A61K 31/19, A61K 31/198, A61P 1/00, A61P 1/04

(54) **COMPOSITIONS COMPRISING AMINO ACIDS FOR USE IN THE PREVENTION AND/OR TREATMENT OF INTESTINAL DISEASES**

(30) Priority: 23.06.2022 IT 202200013354
(71) Applicant: Professional Dietetics S.p.A., 20129 Milano (MI) (IT)
(72) Inventor: GIORGETTI, Paolo Luca Maria, I-20124 Milano (IT)
(74) Representative: Cesa, Roberta

(57) **Abstract**

Composition for use in the prevention and/or in the treatment of an intestinal disease, the composition comprising an active agent, said active agent containing the amino acids leucine, isoleucine, valine, threonine, lysine and citric acid, succinic acid, malic acid.

## Description

### Field of the invention

The present description relates generally to compositions comprising amino acids for use in the prevention and/or treatment of intestinal diseases, in particular associated with inflammation and altered permeability.

### Background

The intestinal mucosal barrier (IMB) is critical for the absorption of nutrients and the health of both humans and animals. Recent publications from clinical and experimental studies have shown the importance of the nutrients-bacteria-host interaction for intestinal homeostasis. Dysfunction of the nutrients-bacteria-host interactions has been reported to be associated with metabolic disorders and the development of intestinal diseases such as irritable intestinal syndrome and inflammatory intestinal diseases (IBD), ulcerative colitis (UC), and Crohn's disease (CD) being the two major types of IBD. On the other hand, systemic disorders, including obesity and type 2 diabetes, and specific diseases, such as kidney disorders, are linked to IMB dysfunction and loss.

The IBD causes chronic intestinal inflammation resulting in relapsing and remitting symptoms, including abdominal pain, diarrhoea, anaemia and weight loss (Solberg et al., Scand. J. Gastroenterol. 44, 431-440, 2009; Solberg et al. Clin. Gastroenterol. Hepatol. 5, 1430-1438, 2007). The clinical course of IBD is highly variable, both between individuals and during the life of a given individual. Periods of a clinically inactive disease can be disrupted by acute flares, leading to the need for medication, hospitalization and, sometimes, intestinal surgery. The pathogenesis of IBD is driven by an abnormal and prolonged T cell-mediated immune response directed toward the commensal gut microbiota that occurs in genetically susceptible individuals. The known IBD risk genes are related to various immune functions, including innate immune functions such as physical barrier and autophagy. The current models implicate multiple factors in IBD pathogenesis, including multi-layered mucosal injuries such as histological and cellular-level changes that ultimately lead to macroscopic erosions and ulcers. Disruption of the intestinal barrier may follow a depletion of intestinal Tight Junction (TJ) structures that are protein structures localized in the apical portion of enterocytes connecting both epithelial and endothelial cells. The disruption of the intestinal barrier (wounding) may lead to the translocation of microorganisms and other antigens into the intestinal wall with a consequent uncontrolled immune activation as key feature of the IBD (Neurath, M. F. & Travis, S. P. Gut 61, 1619-1635, 2012). The structural basis of a healed mucosa is an intact barrier that limits bacterial translocation and consequent immune activation. Thus, a functional definition of mucosal healing can be described as a mucosa with a re-established barrier function. Almost all currently available treatments for IBD act by inhibiting inflammation, often blocking specific inflammatory molecules. However, given the infectious and neoplastic disease burden associated with chronic immunosuppressive therapy, the goal of attaining mucosal healing without immunosuppression is attractive.

In addition to the physical barrier composed of epithelial cells, the production of proteins, TJ and intracellular mechanisms to deal with invading pathogens is critical to maintaining homeostasis and must be regenerated in the process of mucosal healing; notably, the intestinal epithelium is not static but highly dynamic and renews every 5-7 days, allowing continued protection of immune cells from the rich luminal microbiota. Mucins are glycoproteins produced and secreted by goblet cells and are considered the major component of the mucus layer, which separates the commensal bacteria from the epithelium. Microbial control is a substantial part of the functional barrier; Paneth cells are located at the bottom of the intestinal crypt in the stem cell niche and are the major source of antimicrobial peptides (AMPs).

Over the past 20 years, complete mucosal healing has been identified as an important therapeutic goal in IBD. In the Inflammatory Bowel South-Eastern Norway (IBSEN) study, for example, mucosal healing at the 1-year follow-up visit after diagnosis was associated with a reduced risk of future colectomy in patients with ulcerative colitis and less inflammation after 5 years and decreased future steroid treatment in patients with Crohn's disease (Froslie et al., Gastroenterology 133, 412-422, 2007).

Therapeutic strategies to directly promote mucosal healing are still lacking although an attractive goal is that of favouring mucosal healing without immunosuppression. To this aim, dozens of pathways promoting mucosal healing have been identified; most of them, however, may lead to over-proliferation of the intestinal epithelium and consequently tumour growth.

### Summary of the invention

The present description has the aim of providing amino acid-based compositions particularly effective in preventing and/or treating an intestinal disease in a subject particularly associated with intestinal inflammation and altered permeability, by favouring mucosal healing without exerting an immunosuppressive action and/or an uncontrolled intestinal cell proliferation.

According to the present description, the above object is achieved thanks to the subject matter specifically recalled in the ensuing claims, which are understood as forming an integral part of this disclosure.

An embodiment of the present description provides a composition for preventing and/or treating an intestinal disease in a subject, the composition comprising an active agent, said active agent containing the amino acids leucine, isoleucine, valine, threonine, lysine and citric acid, succinic acid, malic acid or acceptable salts thereof.

The intestinal disease may be selected in the group consisting of irritable intestinal disease (IBS) and inflammatory intestinal disease (IBD), wherein the IBD may be selected between Crohn's disease and ulcerative colitis.

In one or more embodiments, the active agent of the composition may further contain one or more amino acids selected in the group consisting of histidine, phenylalanine, methionine, tryptophan, cysteine, tyrosine.

The disclosure also provides a method for preventing and/or treating an intestinal disease in a subject. The method is capable of preventing and/or treating an intestinal disease selected in the group consisting of irritable intestinal disease (IBS) and inflammatory intestinal disease (IBD), wherein the IBD may be preferably selected between Crohn's disease and ulcerative colitis. The method comprises selecting a composition comprising an active agent, said active agent containing the amino acids leucine, isoleucine, valine, threonine, lysine, and the carboxylic acids citric acid, succinic acid, and malic acid, and administering the composition to the subject. The active agent may further comprise one or more amino acids selected in the group consisting of histidine, phenylalanine, methionine, tryptophan, cysteine, tyrosine, as disclosed herein.

### Brief description of the drawings

The invention will now be described, by way of example only, with reference to the enclosed figures, wherein:

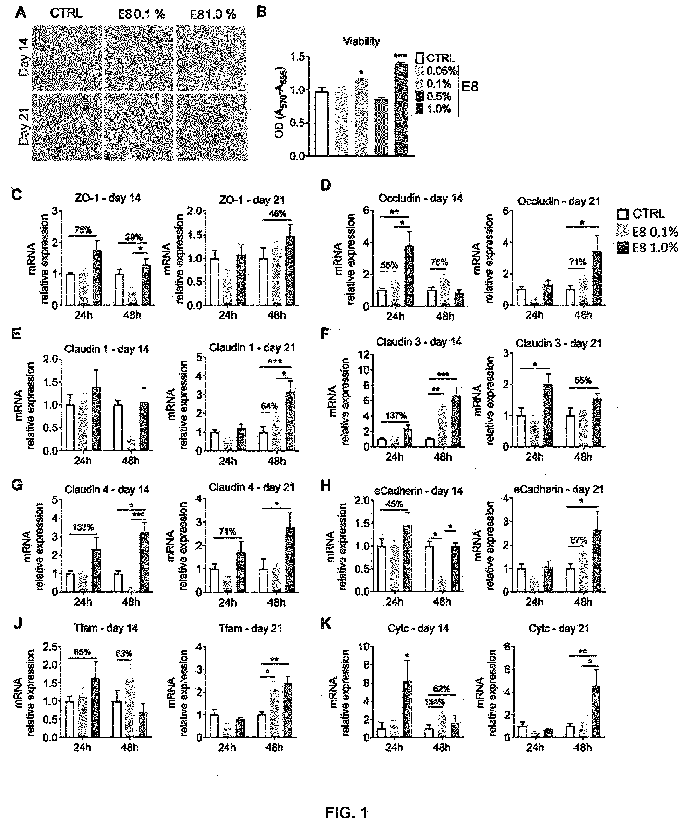

- **Figure 1** shows the effect of E8 on the healthy state of Caco2 cells at different time points of differentiation. (A) Representative image of Caco2 cells differentiated for 14 days or 21 days and treated with E7 (0.1 - 1.0 %) for 24 h (scale 20x); (B) Viability of Caco2 cells differentiated for 21 days and treated for 24h with E8 (0.05-1.0 %) measured by MTT assay; (C-K) mRNA levels of intestinal permeability (C-G) and mitochondrial biogenesis (J,K) genes in Caco2 cells differentiated for 14 or 21 days and treated with E7 (0.1-1.0 %) for 24 or 48 h. The cycle number at which the transcripts were detectable was compared to that of GAPDH and expressed as relative expression versus control (CTRL), taken as 1.0. Data are expressed as mean ± SEM (n = 3-4 samples/group). One-way ANOVA (B), two-way ANOVA (C-K), *p<0.05, **p<0.01, ***p<0.001 vs. CTRL;

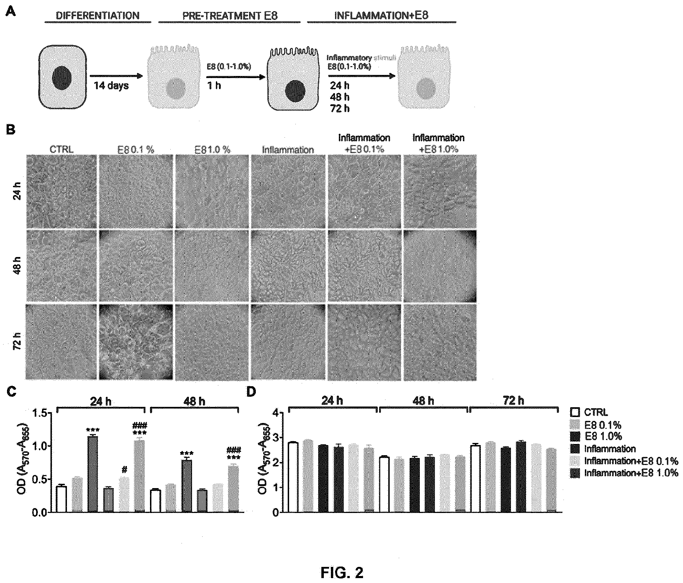

- **Figure 2** shows the effect of E8 treatment on cell viability in the in vitro model of gut inflammation. (A) In vitro model of gut inflammation and E8 treatments; (B) Representative image of Caco2 cells differentiated for 14 days and treated with E7 (0.1 -1.0 %) in the presence or absence of inflammatory stimuli [i.e., IL-1β (25 ng/ml), TNF-α (50 ng/ml) and LPS (10 µg/ml)] for 24, 48, and 72 h (scale 20x); (C, D) Viability measured by MTT (C) and by TOX6 (D) assays. Data are expressed as mean ± SEM (n = 3-4 samples/group). Two-way ANOVA, ***p<0.001 vs CTRL, #p<0.05 and ###p<0.001 vs inflammation;

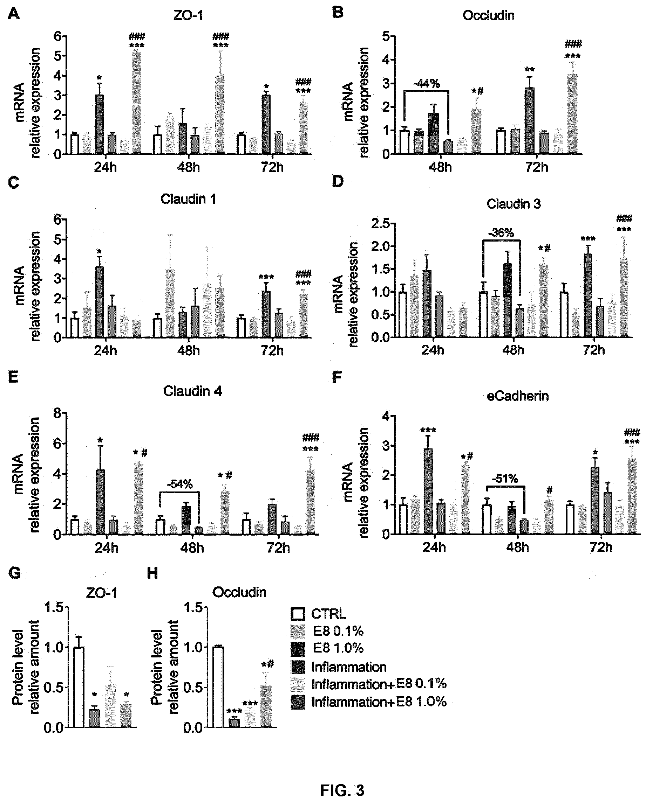

- **Figure 3** shows the effect of E8 treatment on gut permeability in the in vitro model of intestinal inflammation. (A-F) mRNA levels of intestinal permeability markers in Caco2 cells differentiated for 14 days and treated with E8 (0.1-1.0 %) for 24, 48 h, and 72h. The cycle number at which the transcripts were detectable was compared to that of GAPDH and expressed as relative expression versus control (CTRL), taken as 1.0. (G-H) Western Blot analysis of zonulin 1 (ZO-1) and occluding protein level in Caco2 in cells differentiated for 14 days and treated with E7 (0.1-1.0 %) for 48 h. Data are expressed as mean ± SEM (n = 3-4 samples/group). Two-way ANOVA (A-F), one-way ANOVA (G-H), *p<0.05, **p<0.01, ***p<0.001 vs CTRL; #p<0.05 and ###p<0.001 vs inflammation

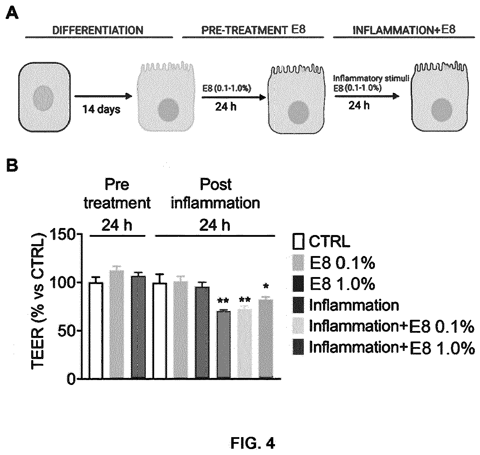

- **Figure 4** shows the effect of E8 treatment on barrier integrity in the in vitro model of gut inflammation. (A) Differentiated CaCo2 cells for 14 days, cultured on 24 well inserts, were pre-treated with E7 (0.1 - 1.0 %) for 24 h and then exposed to inflammatory stimuli for another 24 h. IL-1b (25 ng/ml) and TNF-a (50 ng/ml) were applied at the basolateral side of the cells; LPS (5 µg/ml) was applied on both sides. (B) The TEER was measured at the beginning and the end of the experiment. Values are expressed as a percentage to CTRL and represent means ±SEM, n=3-4. One-way ANOVA, *p<0.05, **p<0.01, vs CTRL;

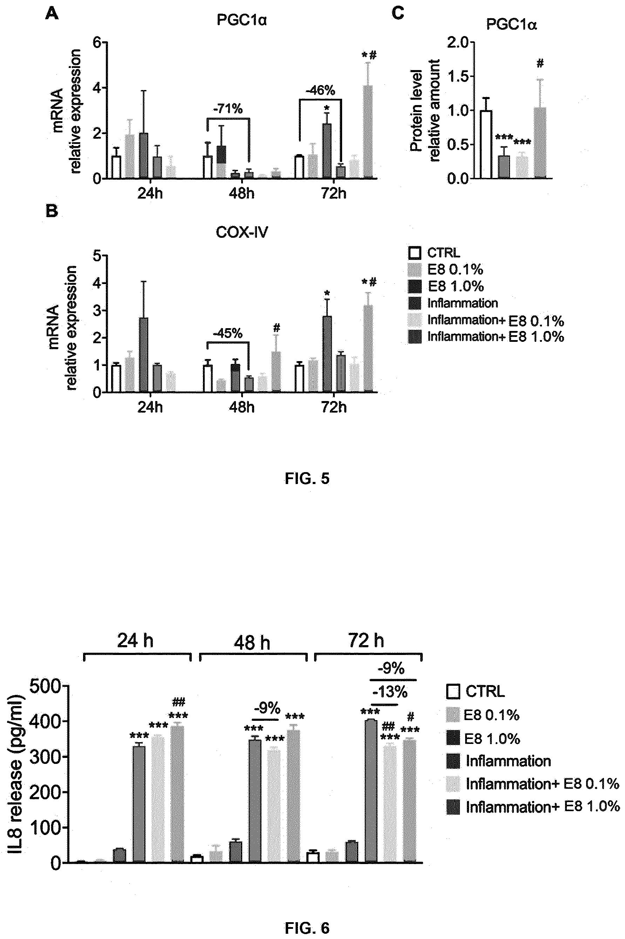

- **Figure 5** shows the effect of E8 treatment on mitochondrial biogenesis in the in vitro model of intestinal inflammation. (A, B) mRNA relative expression of mitochondrial biogenesis markers in Caco2 cells differentiated for 14 days and treated with E7 (0.1-1.0 %) for 24, 48 h, and 72h. The cycle number at which the transcripts were detectable was compared to that of GAPDH and expressed as relative expression versus control (CTRL), taken as 1.0. (C) Western Blot analysis of PGC1α in Caco2 cells differentiated for 14 days and treated with E7 (0.1-1.0 %) for 48 h. Data are expressed as mean ± SEM (n = 3-4 samples/group). Two-way ANOVA, *p<0.05, ***p<0.001 vs CTRL; #p<0.05 vs inflammation;
- **Figure 6** shows the effect of E8 treatment on IL8 secretion in the in vitro model of intestinal inflammation. Caco-2 cells differentiated for 14 days were pre-treated with E7 for 1h and then exposed or not to inflammatory stimuli, as previously reported. IL8 secretion in the culture medium was evaluated by ELISA kit. Values are expressed as pg/ml and represent means ± SEM (n = 3 samples/group). Two-way ANOVA, ***p<0.001, vs CTRL; #p<0.05, and ##p<0.01 vs inflammation;

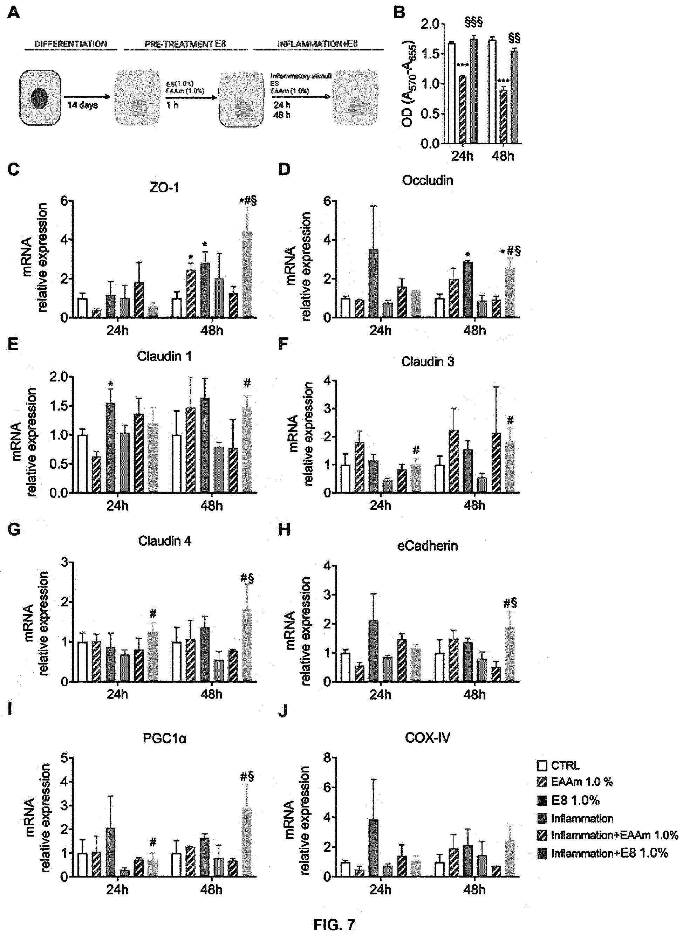

- **Figure 7** shows a comparison of efficacy and safety of E8 and EAAm compositions in the in vitro model of intestinal inflammation. (A) Caco2 cells differentiated for 14 days were pre-treated with E7 (1.0 %) and EAAm (1.0 %), i.e., a composition without TCA cycle intermediates, for 1h and subsequently exposed or not with inflammatory stimuli for 24 or 48 h; (B) Viability of Caco2 cells differentiated for 14 days and treated for 24 and 48 h with E7 or EAAm (1.0 %) measured by MTT assay; (C-J) mRNA relative expression of gut permeability (C-H) and mitochondrial biogenesis (G-H) genes. The cycle number at which the transcripts were detectable was compared to that of GAPDH and expressed as relative expression versus control (CTRL), taken as 1.0. Data are expressed as mean ± SEM (n = 3-4 samples/group). Two-way ANOVA, *p<0.05, ***p<0.001 vs CTRL; #p<0.05 vs inflammation, §p<0.05 vs EAAm treated cells;

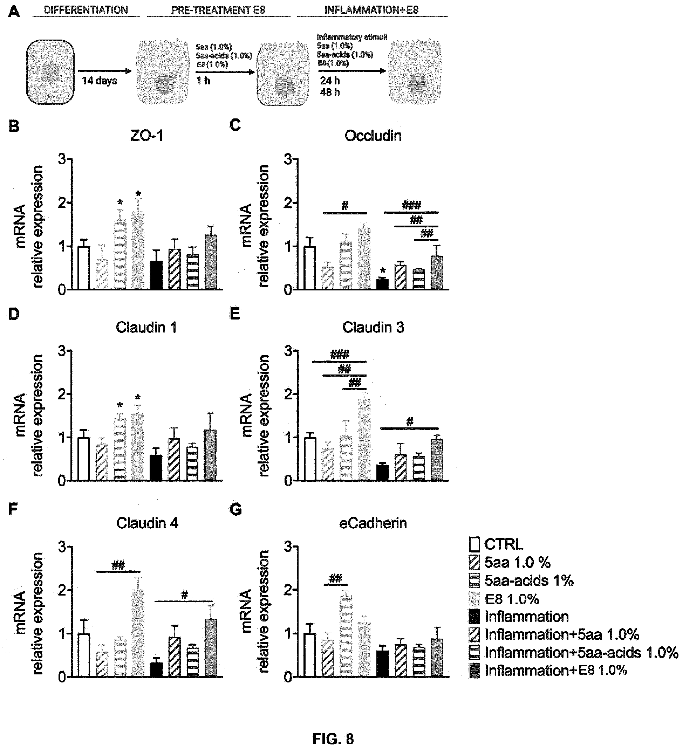

- **Figure 8** shows the effect of 5aa-based compositions in the *in vitro* model of intestinal inflammation. (A) Caco2 cells differentiated for 14 days were pre-treated with 5AA, 5AA-acids, and E7(all 1.0 %) for 1h and exposed or not with inflammatory stimuli for 24h. (A-G) mRNA relative expression of gut permeability genes. The cycle number at which the transcripts were detectable was compared to that of GAPDH and expressed as relative expression versus control (CTRL), taken as 1.0. Data are expressed as mean ± SEM (n = 3-4 samples/group). Two-way ANOVA, *p<0.05 vs CTRL; #p<0.05, ##p<0.01, ###p<0.001 as specifically reported in each panel.

### Detailed description of preferred embodiments

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. The headings provided herein are for convenience only and do not interpret the scope or meaning of the embodiments.

The present description has the aim of providing amino acid-based compositions particularly effective in preventing and/or treating an intestinal disease in a subject. The intestinal disease may be selected in the group consisting of irritable intestinal disease (IBS) and inflammatory intestinal disease (IBD), wherein the IBD may be preferably selected between Crohn's disease and ulcerative colitis.

The compositions for use in preventing and/or treating an intestinal disease in a subject comprises an active agent, said active agent containing the amino acids leucine, isoleucine, valine, threonine, lysine and citric acid, succinic acid, malic acid.

In one or more embodiments, the subject may be a subject affected by chronic kidney disease (CKD) and the intestinal disease may be preferably selected between irritable intestinal disease (IBS) and inflammatory intestinal disease (IBD), wherein the IBD may be selected between Crohn's disease and ulcerative colitis.

The Inventor of the instant application has surprisingly found that the compositions herein disclosed are particularly effective in ameliorating the intestinal inflammation and permeability that represents two mutually conditioning factors in that inflammation with the presence of cytokines disrupts TJs and the dysregulated TJs, in turn, permit intestinal translocation with activation of both local and systemic inflammation.

Specifically, faecal levels of calprotectin, a marker of intestinal inflammation and zonulin, a marker of IMB dysfunction were measured in subjects affected by chronic kidney disease (CKD), the IMB dysfunction being a risk factor for chronic CKD progression. In particular, in CKD a combination of gut inflammation and gut permeability may cause intestinal barrier disruption leading to translocations of lumen bacteria and toxic bacterial by-products (enterotoxins) into systemic circulation (endotoxemia). Endotoxemia is responsible for the development of systemic inflammation, acceleration of CKD progression, uremic syndrome, cardiovascular disease, increased risk of mortality, as documented in both animal and humans with advanced CKD. In CKD, gut dysbiosis, i.e. alterations of intestinal microbiota, is a key factor for gut inflammation. For example, duodenum and jejunum that, under physiologic conditions, are not colonized by bacteria, in CKD are colonized by aerobic and anaerobic bacteria, responsible for chronic inflammation of the gastro-intestinal tract such esophagitis, gastritis, duodenitis, enteritis, colitis. Increased intestinal permeability in patients with CKD follows the disruption and depletion of the intestinal TJ structures caused by elevated azotemia and the disruption and depletion of TJs lead to loss of barrier homeostasis and increased intestinal permeability.

The measures of elevated faecal calprotectin and zonulin in subjects affected by CKD have been elected for the clinical study provided in the following sections since the two markers have been previously used for the diagnosis of intestinal inflammation (Ayling RM Adv Clin Chem 2018 87:161-190) and permeability (Fasano A Clin Gastroenterol Hepatol 2012 10:1096-1100), respectively.

The Inventor of the instant application provides herein the evidence that, compared to non-supplemented healthy subjects, the baseline faecal calprotectin and zonulin were significantly higher in the CKD patients suggesting that nephropathy promoted intestinal inflammation and IMB dysfunction.

After 6 months of supplementation with the composition herein disclosed, calprotectin and zonulin levels significantly decreased compared to their levels before treatment in the faecal sample of CKD patients therefore providing the evidence of the effectiveness of the composition in the treatment of an intestinal disease associated with inflammation and permeability.

The composition administered to the CKD patients comprises an active agent, containing citric acid, succinic acid and malic acid in combination with leucine, isoleucine, valine, threonine, lysine.

The weight ratio between the total amount of citric acid, succinic acid and malic acid and the total amount of the amino acids leucine, isoleucine, valine, threonine, lysine may be comprised between 0.05 and 0.3, preferably between 0.1 and 0.25.

In one or more embodiments, the composition may consist of leucine, isoleucine, valine, threonine, lysine, citric acid, succinic acid and malic acid and optionally vitamin B1 and/or vitamin B6.

In one or more embodiments, the active agent may further comprise one or more amino acids selected in the group consisting of histidine, phenylalanine, methionine, tryptophan, cysteine, tyrosine.

In one or more embodiments, the composition may comprise an active agent consisting of leucine, isoleucine, valine, threonine, lysine, histidine, phenylalanine, methionine, tryptophan, cysteine, tyrosine, as well as citric acid, succinic acid and malic acid, said amino acids being the sole amino acids contained in the composition. In one or more embodiments, the composition does not comprise any further amino acids.

In one or more embodiments, the composition may consist of leucine, isoleucine, valine, threonine, lysine, histidine, phenylalanine, methionine, tryptophan, cysteine, citric acid, succinic acid and malic acid and optionally vitamin B1 and/or vitamin B6.

The composition may comprise the amino acids isoleucine, leucine and valine in an amount between 35% and 65% by weight, preferably between 42% and 58% by weight with respect to the composition weight.

The composition may comprise the amino acids isoleucine, leucine, valine, lysine and threonine in an amount between 50% and 95% by weight, preferably between 65% and 90% by weight with respect to the composition weight. The composition may comprise citric acid, malic acid, succinic acid in an amount between 5% and 50% by weight, preferably between 8% and 35% by weight with respect to the composition weight.

The weight ratio between leucine and citric acid may be comprised between 1 and 5, preferably between 2.50 and 3.90.

In one or more embodiments, the weight or molar amount of citric acid is higher than the weight or molar amount of each of malic acid and succinic acid. Preferably, the weight or molar amount of citric acid is higher than the weight or molar overall amount of malic acid plus succinic acid. In a further embodiment, the weight ratio between citric acid and the sum of malic acid and succinic acid is comprised between 1.0 and 4.0, preferably between 1.5 and 2.5. In a preferred embodiment, the citric acid:malic acid:succinic acid weight ratio is comprised between 10:1:1 and 2:1.5:1.5, preferably between 7:1:1 and 1.5:1:1, more preferably between 5:1:1 and 3:1:1. In a preferred embodiment the citric acid:malic acid:succinic acid weight ratio is 4:1:1.

The preferred isoleucine:leucine weight ratio is comprised in the range 0.2-0.7, preferably in the range 0.30-0.60 and/or the preferred valine:leucine weight ratio is comprised in the range 0.2-0.70, preferably in the range 0.30-0.65.

The threonine:leucine weight ratio may be comprised in the range of 0.10-0.90, preferably in the range 0.20-0.70 and/or the lysine:leucine weight ratio is comprised in the range of 0.20-1.00, preferably in the range 0.40-0.90.

In a preferred embodiment, the ratio between the overall weight amount of citric acid, malic acid, succinic acid and the overall weight amount of methionine, phenylalanine, histidine and tryptophan is higher than 1.35.

In one or more embodiments, the weight ratio between the sum of citric acid, malic acid, succinic acid and the sum of the branched-chain amino acids leucine, isoleucine, valine is comprised between 0.1 and 0.4, preferably between 0.15 and 0.35.

In a further embodiment, the overall weight amount of the branched-chain amino acids leucine, isoleucine, valine plus threonine and lysine is higher than the overall weight amount of the three acids citric acid, malic acid, succinic acid. Preferably, the weight amount of the single acids (citric acid, succinic acid or malic acid) is less than the weight amount of each of the single amino acids leucine, isoleucine, valine, threonine and lysine.

In a further embodiment, the overall weight amount of lysine and threonine is higher than the overall weight amount of the three acids citric acid, succinic acid, malic acid. Preferably, the ratio between the overall weight amount of the three acids citric acid, succinic acid, malic acid and the overall weight amount of lysine and threonine is comprised between 0.1 and 0.7, preferably between 0.15 and 0.55.

In one or more embodiments, the amino acids are present in the composition in free form. An advantage linked to the use of the composition described herein lies in the fact that the use of amino acids in free form comprised in the active agent allows producing such compositions at a comparatively extremely low cost with respect to synthetic proteins and growth factors, through per se known production processes and widely used in the field of preparing compositions based on free amino acids. The field of application of the invention may however also be extended to amino acids obtained through genetic engineering or any other artificial method.

In one or more embodiments, the composition herein disclosed may further comprise vitamins, preferably selected in the group of vitamins B, such as vitamin B₁ and/or vitamin B₆. The vitamins may be included in the composition in an amount higher than 0,001%, preferably comprised between 0.001 and 0.5% by weight of the composition.

In addition, the composition may further include carbohydrates and/or flavouring substances.

In one or more embodiments, the composition may be a pharmaceutical composition further comprising a pharmaceutically acceptable vehicle. The composition may also comprise pharmaceutically acceptable excipients, like for example vitamins, carbohydrates, natural and artificial sweeteners and/or flavoring substances. In a preferred embodiment, the pharmaceutically acceptable excipients may be selected from maltodextrins, fructose, fish oil, sucralose, sucrose esters, vitamin D3.

Furthermore, in particular when preparing the compositions according to the instant disclosure, and specifically the active agent, the amino acid arginine is preferably to be avoided. In addition, further amino acids that may be specifically excluded by the composition herein disclosed are serine, proline, alanine. Such amino acids can be counterproductive or even harmful in some concentrations or stoichiometric ratios within the composition.

The amino acids disclosed in the instant description may be replaced by respective pharmaceutically acceptable derivatives, namely salts. The carboxylic acids of the active agent may be replaced by respective pharmaceutically acceptable derivatives, namely salts.

For oral use, the compositions according to the description may be in the form of tablets, capsules, granules, gel, jellifying powder, powder.

The disclosure also provides a method for preventing and/or treating a intestinal disease in a subject. The intestinal disease may be selected in the group consisting of irritable intestinal disease (IBS) and inflammatory intestinal disease (IBD), wherein the IBD may be preferably selected between Crohn's disease and ulcerative colitis. The method comprises selecting a composition comprising an active agent, said active agent containing the amino acids leucine, isoleucine, valine, threonine, lysine, and the carboxylic acids citric acid, succinic acid, and malic acid, and administering the composition to the subject. The active agent may further comprise one or more amino acids selected in the group consisting of histidine, phenylalanine, methionine, tryptophan, cysteine, tyrosine, as disclosed herein.

Further specifications, in terms of amounts and ratios among the various amino acids provided for by the compositions are contained in the attached claims, which form an integral part of the technical teaching provided herein in relation to the invention.

### EXAMPLES

Examples of compositions of the present disclosure are shown in the following Table 1.

**Table 1**

| **Composition (% w/w)** | **α5** | **α7** | **E7** |
|---|---|---|---|
| | | | |
| L-Leucine | 31.0885 | 27.7256 | 23.4579 |
| L-Lysine HCl chlorhydrate | 16.9030 | 17.2659 | 17.5930 |
| L-Isoleucine | 10.3628 | 13.8628 | 11.7290 |
| L-Valine | 10.3628 | 13.8628 | 11.7290 |
| L-Threonine | 7.2540 | 12.2751 | 13.6840 |
| L-Cysteine | 3.1089 | - | 2.9320 |
| L-Histidine | 3.1089 | - | 2.9320 |
| L-Phenylalanine | 2.0726 | - | 1.9550 |
| L-Methionine | 1.0363 | - | 0.9774 |
| L-Tyrosine | 0.6218 | - | |
| L-Tryptophan | 2.0726 | - | 0.9774 |
| Citric acid | 8.001 | 10.00 | 7.9950 |
| Succinic acid | 2.00 | 2.5 | 2.0040 |
| Malic acid | 2.00 | 2.5 | 2.0040 |
| Vitamin B1 (thiamine chlorhydrate) | 0.004 | 0.004 | 0.0166 |
| Vitamin B6 (piridoxine chlorhydrate) | 0.0038 | 0.0038 | 0.0137 |

The compositions of Table 1 above may be prepared first by sifting all the components with a 0.8 mesh. To obtain a pre-mixture, each ingredient (in an amount <10% by weight of the total amount) is put in a polyethylene bag together with a portion of L-lysine HCl so as to obtain about 10% of the weight of the total composition. The bag is then manually shaken for 5 minutes. The pre-mixture is then loaded in a mixer (Planetaria) together with the remainder of the ingredients and mixed for a period of 15 minutes at 120 rpm to obtain a homogeneous final composition.

### METHODS - part 1

### Patients

The study was approved by the Ethics Committee of the Local Health Authorities (Reg 74/2018) after the entire study population provided their written informed consent.

Twenty-nine elderly (age>65 years) patients had been preselected for having a diagnosis of stage 3b-4 CKD (estimated glomerular filtration-eGFR- rate between 44-29 ml/min/1.73 m²). The reason for choosing elderly subjects lies in the higher possibility to find alterations of gut barrier integrity as ageing is associated with loss of TJ, microbial dysbiosis, intestinal barrier dysfunction. Among these patients those with concomitant acute and chronic inflammatory disease (n=3), diabetes (n=5), cancer (n=0), autoimmune diseases (n=1), on steroid/immunosuppressant treatment (n=0), chronic obstructive pulmonary disease (n=7), heart failure (n=2), liver diseases (n=1), were excluded. Moreover, cigarette smoker patients were excluded as cigarette smocking is associated with altered intestinal tight junctions. Therefore, of initial 29 patients, 9 patients were investigated. The patients were on diet treatment with protein intakes of 0.6-0.7 g/kg (50% of animal origin), caloric intake >30 Kcal/kg/day, phosphate intake <1000 mg/day. In addition to eGFR, renal function was evaluated by means of the prediction modification of diet in renal disease study group (MDRD) (Menon V Am J Kidney dis 2099 53:208-217) that was normalized to the body surface area.

These patients were asked to undergo a 6-month supplementation with the composition herein disclosed and shown in Table 1 as "composition E7". Measures taken after 12 months of survey served as baseline values for the study. Eleven healthy controls were also enrolled for the study as control group (CTRL).

After enrolment, the patients underwent the following measures:
*1. Biohumoral variables, including 24-hr urine protein content. Urea Nitrogen* Appearance (UNA) was calculated to estimate protein intake (Maroni BJ Kidney Int 1985 27:58-65; Masud T Kidney Int 2002 62:1750-56)
*2. Body composition.* Total Body Water (TBW), Extracellular Water (ECW), Intracellular Water (ICW), all in litres (l) and percentage of body weight, Resistance (RZ in Ohm) Conductance (XC in Ohm), phase angle (in degree), considered an indicator of skeletal muscle mass (Sunario J J Parent Enter Nutr 2021 45:1089-1099) were measured by Bioletrical Impedance Analysis (BIA) by the same operator using the same instruments (renal EFG 50 Hz,; EFG diagnostic Ltd, Belfast Northern Ireland).

### 3. Determination of plasma AAs

Free amino acid concentrations in plasma of supplemented and control patients were measured by cation-exchange chromatography and detected spectrophotometrically (570 nm and 440 nm) after post-column reaction with ninhydrin reagent on a Biochrom 30+ Amino Acid Analyzer (Biochrom Ltd, ERRECI s.r.l Pieve Emanuele, Italy) (n = 8-9 samples/group). The plasma samples were mixed 1:1 in a 5-sulphosalicylic acid 5% (SSA)-based deproteinization reagent - containing L-Norleucine (Sigma Aldrich, Milan, Italy) as the internal standard (C_{f} = 250 mmol/L) - and incubated 30 min at 4 °C. After centrifugation (10,000xg for 5 min at 4 °C), the supernatants were collected and filtered in filter columns (0.22 mm) (Millipore, Milan, Italy) to completely remove proteins (12,000xg for 10 min at 4 °C). The calibration standard (C_{f} of each amino acids = 250 mmol/L) (Biochrom Ltd, Cambridge Research Park, UK) solution was treated with L-Norleucine SSA 5%-based buffer as the samples, to ensure equal pH upon loading. The standard solution and the samples kept cold during the experiment were injected into the analyzer (60 ml/sample). Amino acid separation was achieved by a cation exchange chromatography process. Amino acids concentrations were detected spectrophotometrically after post-column reaction with ninhydrin, under control reaction conditions at 135°C.

### 4. Determination of faecal calprotectin and zonulin levels

Calprotectin determination in stool samples was performed by an immunoenzymatic method and measured by Chrous TRIO instrument (DIESSE Diagnostica Senese S.p:A., Italy) according to the manufacturer's instruction. Calprotectin values <50 µg/g per stool sample were considered normal.

Intestinal permeability was evaluated as a faecal zonulin concentration (ng/ml) using commercial ELISA kit (Zonulin Stool ELISA, DRG Instruments Gmbh, Germany). The normal amount of zonulin in feces of healthy subjects is considered to be <60 ng/ml.

Eleven elderly, healthy non-smoker subjects, matched for age, sex, Body Mass Index (BMI, Kg/m²) served as controls (CTRL). The CTRL underwent the same procedures as CKD but only at the time of their recruitment.

After completing the above measures only patients were prescribed the composition; specifically, the patients were instructed to consume 2 packets/day for 6 months (1 at morning and 1 in the afternoon, diluted in 150-200 ml of water). The composition of each packet is shown in Table 2.

**Table 2**

| **Ingredient** | **mg/packet** |
|---|---|
| L- Leucine | 1200.00 |
| L-Isoleucine | 600.00 |
| L-Valine | 600.00 |
| L-Lysine | 900.00 |
| L-Threonine | 700.00 |
| L-Histidine | 150.00 |
| L-Phenylalanine | 100.00 |
| L-Methionine | 50.00 |
| L-Tryptophan | 50.00 |
| L-Cysteine | 150.00 |
| Citric acid | 409.00 |
| Succinic acid | 102.5 |
| Malic acid | 102.5 |
| Vit. B6 | 0.85 |
| Vit. B 1 | 0.70 |

All the above procedures were repeated in CKD patients after 6 months of composition supplementation.

### 5. Evaluation of patient adherence to the composition

Patients were considered adherent to the prescribed composition if, after over might fasting, at 6 months from initiated supplementation, their plasma glutamine and alanine resulted significantly increased in relation to baseline values. Increased plasma glutamine and alanine levels would follow increased muscle utilization of Branched Chain Amino Acid (BCAA; contained in the composition). Moreover, as a confirmation of the BCAA-induced glutamine and alanine formations, an increase of plasma levels of two ratios was added: glutamine/ (BCAA + aspartic acid + asparagine + glutamic acid) and alanine/(BCAA + aspartic acid + asparagine + glutamic acid). Indeed, also aspartic acid, asparagine, glutamic acid contributes to glutamine and alanine formations.

### 6. Estimation of the effects of the composition on ureagenesis

Blood urea nitrogen (BUN) was evaluated with the Biochrom 30+ Amino Acid Analyzer. This measurement may be especially important in case of progression of GFR, in order to distinguish if a possible increase of BUN was due to increase nitrogen intake with composition supplementation and/or to worsened renal dysfunction. Non-increased or reduction of BUN/plasma total amino acids (AAs) ratio in fasting patients pointed to the absence of nitrogen intake overload.

### 7. Statistical Methods

Central tendency and dispersion of continuous variables are reported as mean ± standard deviation. Discrete variables are reported as numbers (N) and frequency percentage. Several variables violated the normality assumption (Shapiro-Wilk test), hence non-parametric statistics were preferred for hypothesis testing. Within- and between-group comparisons for continuous variables were carried out by the Wilcoxon signed rank test and by the Mann-Whitney U-test, respectively. Categorical variables were compared by the Chi-square test or Fisher's exact test if appropriate. All tests were two-tailed. A p value <0.05 was considered statistically significant. All statistical analyses were carried out using the SAS/STAT statistical package, release 9.4 (SAS Institute Inc., Cary, NC, USA).

### RESULTS - part 1

### Patients' baseline characteristics

Table 3 refers to baseline characteristics of controls (CTR) and Chronic Kidney Disease (CKD) patients. The reported p-values are from Mann-Whitney U-test or *Fisher's Exact Test. CKD patient and CTRL had similar age, Body Weight (as BMI) but different body composition. Indeed the patients showed increased Total Body Weight (TBW; p<0.05) and Extracellular Body Weight (EBW); p<0.01), reduced phase angle (p<0.01) considered an indicator of decreased skeletal muscle mass.

When compared to controls, CKD showed a mild anaemia with lower serum levels of iron (p<0.01) and ferritin (p<0.05) whose mean values were still within the normal range of values of our laboratory, hyperparathyroidism.

From renal standpoint, the patients exhibited a moderate-severe renal failure (MDRD 30.83±7.88 ml/min/1.73 m²) and a compensated metabolic acidosis (reduced plasma bicarbonate levels, p<0.01 in comparison to controls).

Blood Urea Nitrogen (BUN) was higher than in controls (p<0.001) whereas protein intake from UNA was lower (p<0.05) but in absolute values higher than that prescribed (0.7-0.6g/kg/d).

**Table 3**

| | CTR | CKD | p |
|---|---|---|---|
| Gender Male N (%) | 4 (36%) | 4 (50%) | 0.66* |
| Age (years) | 72.27±3.74 | 74.56±6.90 | 0.32 |
| Weight (Kg) | 64.50±12.80 | 62.76±9.05 | 0.61 |
| Body surface (UM) | 1.72±0.20 | 1.66±0.15 | 0.40 |
| fatt_corr_sup_corp | 1.01±0.10 | 1.05±0.09 | 0.40 |
| Rz (Ohm) | 536.3±40.0 | 515.5±113.2 | 0.99 |
| Xc (Ohm) | 52.15±12.42 | 42.25±17.38 | 0.17 |
| Phase angle (degrees) | 5.16±0.60 | 4.22±0.67 | 0.008 |
| Total Body Water (%) | 35.65±16.60 | 55.93±9.20 | 0.012 |
| Extracellular Body Water (%) | 19.2518.48 | 55.75±4.39 | <0.0001 |
| ICW (%) | 44.65±4.10 | 40.69±10.06 | 0.59 |
| Fat mass (Kg) | 21.91±2.40 | 19.97±9.96 | 0.48 |
| Lean mass (Kg) | 27.55±2.85 | 44.00±10.03 | 0.00011 |
| Cell mass (Kg) | 24 86±2 79 | 19.84±6.52 | 0.065 |
| Muscle mass (Kg) | 27.55±2.85 | 24.21±6.07 | 0.32 |
| BMI (Kgm-2) | 24.86±2.79 | 25.31±2.31 | 0.50 |
| Basal Met | 1385±68 | 1299:145 | 0.44 |
| MDRD | 88.20±16.59 | 30.83±7.88 | <0.0001 |
| Hb | 14.16±1.20 | 11.85±1.12 | 0.0006 |
| Albuminemia (g/dL) | 4.21±0.23 | 4.13±0.18 | 0.43 |
| sideremia_(ug/dL) | 106.09:26.69 | 72.38±32.76 | 0.005 |
| Ferritina (ng/mL) | 175.60±93.28 | 78.97±60.65, | 0.016 |
| BUN (mg/dL) | 11.47±3.52 | 43.86±16.09 | <0.0001 |

Table 4 shows baseline measures associated with intestinal mucosa inflammation (Calprotectin) and permeability (Zonulin) of controls (CTR) and Chronic Kidney Disease (CKD) patients. The reported p-values are from Mann-Whitney U-test. The patients showed a state of intestinal mucosa inflammation (higher fecal calprotectin, p=0.005 vs controls) and higher permeability (higher faecal zonulin concentrations, p<0.001 vs controls).

**Table 4**

| | CTR | CKD | p |
|---|---|---|---|
| Calprotectin | 30.25±27.62 | 95.60±53.26 | 0.005 |
| Zonulin | 54.96±32.73 | 219.38±171.50 | 0.001 |

### Changes of baseline variables after 6 months of E7 supplementation (T6-T12)

After 6 months supplementation with the composition, the faecal levels of both calprotectin and zonulin resulted significantly decreased as shown in Table 5 (wherein the p-values are from Wilcoxon signed rank test), indicating reduced intestinal inflammation and permeability.

**Table 5**

| Variable | Baseline | After 6 months AA | p |
|---|---|---|---|
| Calprotectin | 95.60±53.26 | 37.92±22.04 | 0.008 |
| Zonulin | 219.4±171.5 | 70.5±35.9 | 0.05 |

The patients with CKD had a good adherence to the prescribed composition supplementation as showed by increased glutamine/(BCAA + aspartic acid + asparagine + glutamate) ratio (p=0.008) and alanine/(BCAA+aspartic acid+asparagine + glutamate) ratio (p=0.016) (table 6).

**Table 6**

| Variable | Baseline | After 6 months AA | p value |
|---|---|---|---|
| Gln/(BCAA+Asp+Asn+Glu) | 0.43±0.23 | 0.91±0.29 | 0.008 |
| Ala/(BCAA+Asp+Asn+Glu) | 0.61±0.08 | 0.88±0.25 | 0.008 |

Table 7 below shows Bun values, Blood Urea Nitrogen/Total amino acid (AA) ratio and Blood Urea Nitrogen/non essential amino acid (non essential AA) ratio in CKD patients at baseline and after 6 months of supplementation with the composition. The composition did not give rise to increased urea formation given that BUN/Tot AAs was similar to baseline value.

**Table 7**

| Variable | Baseline | After 6 months AA | p value |
|---|---|---|---|
| Bun | 43.86±16.09 | 50.33±9.69 | 0.20 |
| Bun /(Total AA) | 0.020±0.012 | 0.018±0.006 | 0.84 |
| Bun /(Non essential AA) | 0.030±0.018 | 0.025±0.009 | 0.55 |

The results herein provided therefore compared i) the differences in the measured variables between CKD patients before supplementation and the CTRL group and ii) the changes in variables of the CKD patients at baseline and 6 months after the composition supplementation.

The study shows that elderly patients with CKD has reduced intestinal integrity because of inflammation and TJ depletion which increases intestinal permeability; the results indicate, moreover, a good patients' adherence to the composition supplementation which was associated with improved intestinal inflammation and permeability.

The mitochondrial intermediates (malic acid, succinic acid, citric acid) of the composition may have promoted a more balanced inflammatory/adaptive immune response towards adaptive immunity, synergically acting with the amino acids of the composition.

These findings highlight the beneficial effects of the compositions on IMB integrity in patients with intestinal barrier damage or insufficiency, which may originate from gastrointestinal illnesses or other disorders, including dysbiosis, systemic inflammation, CKD, or in critically ill subjects in the intensive care unit.

In the following sections, experimental results are provided in order to analyse a comparison on the effect of different compositions; specifically, the analysis has been carried out to show the synergic effect achieved by the compositions when the three claimed acids (succinic, citric, malic acids) are administered in combination with the claimed amino acids.

### METHODS - part 2

### Cell culture and treatments

Human colon adenocarcinoma Caco2 cells were purchased from the American Type Culture Collection (ATCC-HTB-37, LGC Milan, Italy). Caco2 cells were routinely cultured in standard medium: EMEM (ATCC 30-2003, LGC), supplemented with 10% fetal bovine serum (FBS, ATCC 30-2020, LGC), penicillin (100 U/ml), and streptomycin (100 µg/ml) (Euroclone, Milan, Italy), in a controlled atmosphere with 5% CO2 at 37°C.

For each experiment, confluent Caco2 cells (passages from 10 to 20) were grown in a standard medium for 14 days to obtain fully differentiated cells (i.e., spontaneously form the tight junction between cells and possess brush borders) (Wang et al. 2011). Differentiated Caco2 cells were pre-treated for 1h with the following four compositions: 1) "E8", an essential amino acid composition enriched with tricarboxylic acid (TCA) cycle intermediates (i.e., citric, succinic, and malic acids, ratio 4:1:1) according to embodiments of the instant application, 0.1 and 1.0 % (p/v); 2) "EAAm", an essential amino acid composition without TCA cycle intermediates, 1.0 % (p/v); 3) "5aa", a composition comprising the five claimed amino acids only (i.e., leucine, lysine, isoleucine, valine, and threonine), 1.0 % (p/v); 4) "5aa-acids", the composition "5aa" enriched with TCA intermediates as in E8 (i.e., citric, succinic, and malic acids, ratio 4:1:1) according to embodiments of the instant application, 1.0 % (see Table 8 for the compositions). At the end of pre-treatment, all the conditions were tested in the presence or absence of the inflammatory mediators IL-1β (25 ng/ml), TNF-α (50 ng/ml), and LPS (10 µg/ml) and cultured in EMEM supplemented with 1% heat-inactivated FBS, penicillin (100 U/ml), and streptomycin (100 µg/ml) for 24, 48 or 72 h (Van De Valle J et al. 2010).

**Table 8**

| **Amino acids** | **E8** | **EAAm** | **5aa** | **5aa-acids** |
|---|---|---|---|---|
| L-Leucine | 22.45 | 30.01 | 22.45 | 22.45⁵ |
| L-Lysine (chlorhydrate) | 21.13 | 19.58 | 21.13 | 21.13 |
| L-Isoleucine | 11.23 | 15.00 | 11.23 | 11.23 |
| L-Valine | 11.23 | 15.00 | 11.23 | 11.23 |
| L-Threonine | 13.1 | 8.40 | 13.1 | 13.10 |
| L-Cysteine | 2.81 | 3.60 | - | - |
| L-Histidine | 2.81 | 3.60 | - | - |
| L-Phenylalanine | 1.87 | 2.40 | - | - |
| L-Methionine | 0.94 | 1.20 | - | - |
| L-Tyrosine | - | 0.72 | - | - |
| L-Tryptophan | 0.94 | 0.48 | - | - |
| Vitamin B1 (thiamine chlorhydrate) | 0.02 | - | - | - |
| Vitamin B6 (pyridoxine chlorhydrate) | 0.02 | - | - | - |

| **TCA intermediates** | | | | |
|---|---|---|---|---|
| Citric acid | 7.65 | - | - | 7.65 |
| Malic acid | 1.92 | - | - | 1.92 |
| Succinic acid | 1.92 | - | - | 1.92 |

All values are reported as percentage (g/100 g).

### Gene expression analysis

Total RNA was isolated from Caco2 cells using RNeasy Mini Kit (Qiagen, Milan, Italy) and treated with DNase according to the manufacturer's protocol (Bio-Rad Laboratories, Milan, Italy). cDNA was synthesized using an iScript cDNA Synthesis Kit (Bio-Rad Laboratories) and amplified by real-time quantitative PCR with iTaq Universal SYBR Green SuperMix (BioRad Laboratories) on a CFX Connect Real-Time PCR System (Bio-Rad Laboratories). Primers were designed using Primer3 (version 0.4.0) software (Table 9). The cycle number at which the various transcripts were detectable (threshold cycle, CT) was compared with housekeeping CT, referred to as ΔCT. The gene relative level was expressed as 2^{-ΔΔCT}, in which ΔΔCT corresponded to the difference between the ΔCT of either treatment group and the ΔCT of the control group (Ruocco et al. 2020).

**Table 9**

| **Gene** | **Primer Sense (5'-3')** | **Primer Antisense (5'-3')** | **PCR Product (bp)** | **Tₐ (°C)** | **Seq ID** |
|---|---|---|---|---|---|
| *Claudin 1* | ccgttggcatgaagtgtatg | ccagtgaagagagcctgacc | 208 | 60 | 1, 2 |
| *Claudin 3* | aaggtgtacgactcgctgct | agtcccggataatggtgttg | 247 | 60 | 3, 4 |
| *Claudin 4* | gtctgcctgcatctcctctgt | cctctaaacccgtccatcca | 149 | 60 | 5, 6 |
| *COX-IV* | ccagaaggcattgaaggaga | gggccgtacacatagtgctt | 210 | 60 | 7, 8 |
| *Cyt c* | cgttgtgccagcgactaaaaa | ttccgcccaaagagacca | 151 | 60 | 9, 10 |
| *eCadherin* | tggaccgagagagtttccct | cccttgtacgtggtgggatt | 148 | 60 | 11, 12 |
| *GAPDH* | ggctgagaacgggaagcttgt | ccagcatcgccccacttgat | 90 | 60 | 13, 14 |
| *Occludin* | gaagccaaacctctgtgagc | gaagacatcgtctggggtgt | 229 | 60 | 15, 16 |
| *PGC1α* | agctgctgaagaggcaagag | ttcccctaaaccaagcacac | 163 | 60 | 17, 18 |
| *Tfam* | agattggggtcgggtcac | gacaacttgccaagacagatg | 185 | 60 | 19, 20 |
| *Zonulin 1* | gaatgatggttggtatggtgcg | tcagaagtgtgtctactgtccg | 192 | 60 | 21, 22 |

| | | | | | |
|---|---|---|---|---|---|
| Tₐ, temperature of annealing; COX-IV, Cytochrome c oxidase (complex IV); Cyt c, cytocrome c; GAPDH, glyceraldehyde 3-phosphate dehydrogenase; PGC1α, Peroxisome proliferator-activated receptor-gamma coactivator (PGC)-1alpha; Tfam, mitochondrial transcription factor A. | | | | | |

### Immunoblot analysis

Protein extracts were obtained from differentiated Caco2 cells using Mammalian Protein Extraction Reagent (M-PER, Pierce, Thermo Fisher Scientific, Merck, Milan, Italy), as indicated by the manufacturer, in the presence of a protease and phosphatase inhibitor cocktail (Sigma-Aldrich, Merck, Milan, Italy). Protein content was determined with bicinchoninic acid protein assays (BCA, Euroclone, Milan, Italy). An appropriate amount of protein was run on sodium dodecyl sulfate-polyacrylamide gel electrophoresis (SDS-PAGE) under reducing conditions for immunoblotting. The separated proteins were then semi-dry transferred to a nitrocellulose membrane (Bio-Rad Laboratories), and proteins of interest were revealed with specific antibodies: anti-Zonulin1 and anti-Occludin (all from Invitrogen, Thermo Fisher Scientific) and anti-PGC-1a (Abcam, Prodotti Gianni, Milan, Italy) each at 1:1,000 dilution. Anti-Vinculin (1:3,000; Sigma-Aldrich, Milan, Italy) was used as the loading control. Immunostaining was detected using horseradish peroxidase-conjugated anti-rabbit or anti-mouse immunoglobulin (Corsetti et al. 2014). Amounts of each protein were measured using SuperSignal Substrate (Euroclone), analysed with Chemidoc XRS+, and quantified by ImageLab software (both from Bio-Rad Laboratories).

### Cell vitality assay

The differentiated Caco2 cell viability was determined using the standard MTT [3-(4,5-dimethylthiazol-2-yl)-2,5-diphenyltetrazolium bromide] assay. The MTT is a yellow dye, which is reduced by multiple oxidoreductases, including the mitochondrial succinate dehydrogenase, to the blue product formazan. Differentiated Caco2 cells, seeded in 96-well plates (100 µl/wells), were treated with each composition in the presence or absence of inflammatory mediators for 24 h and 48 h. At the end of the treatments, MTT (5 mg/ml in PBS; 20 µl) was added and further incubated for 4 h. The purple formazan crystals were dissolved overnight at 37°C in 5% SDS/0.1 M HCl (100 µL/well), and the absorbance was recorded on a microplate reader at a dual-wavelength of 570 nm/655 nm (Ragni et al. 2022). Alternatively, cell viability was determined using Sulforhodamine B based in vitro Toxicology Assay Kit (TOX6, Sigma Aldrich), following the manufacturer's instruction.

### IL-8 secretion in cell culture supernatants

For the evaluation of IL8 secretion by differentiated Caco2 cells treated with E8 composition in the presence or absence of inflammatory modulators for 24, 48, and 72 h, the extracellular media were collected and centrifuged at 2,000 g for 10 min. IL-8 release was evaluated using a sandwich ELISA method (ab214030 - Human IL-8 ELISA Kit, Abcam) and quantified in pg/ml using the standard provided with the kit.

### Intestinal barrier integrity

Caco2 cells were seeded on polycarbonate membrane transwell inserts with a pore diameter of 0.4 µm (Brand, Sigma Aldrich) at 1 x 105 cells/well density. When cells reached the confluence, they were grown for 14 days to obtain differentiated Caco2 cells. Cell culture inserts allow access to both the basolateral and the apical sides of the cells, representative of the circulatory and luminal poles of the intestinal epithelium respectively. Differentiated Caco2 cells were pre-treated with the E8 composition (0.1 and 1.0%) at the apical side of cells, and after 24 h, cells were exposed or not to inflammatory stimuli. The IL-1β (25 ng/ml) and TNF-α (100 ng/ml) were applied at the basolateral side of the cells; in contrast, LPS was applied at both sides (5 µg/ml/side), representing, on the one hand, the typical luminal microflora and, on the other hand, the increased presence of bacterial components in serum during inflammatory diseases (Van De Valle J et al. 2010). The trans-epithelial electrical resistance (TEER) was measured at the beginning, after 24h pre-treatment, and at the end of the experiment using the Epithelial Volt/Ohm Meter EVOM3 (World Precision Instruments, MatTek In Vitro Life Science Laboratories, Slovak Republic) (Hiebl et al. 2020).

### Statistical analyses

The sample size of each experiment reflects the number of independent biological replicates, as provided in the figure legends. Data are reported as mean ± SEM. Statistical analyses were performed by one-way or two-way ANOVA test using the Prism 6.0 software (GraphPad Software, Inc.). A value of p< 0.05 was considered statistically significant.

### RESULTS - part 2

### Composition E8 improved intestinal permeability and mitochondrial biogenesis in Caco2 in vitro model of intestinal barrier

The human epithelial cell line Caco2, isolated from a human colorectal adenocarcinoma by Fogh & Trempe in 1974, is the cell line most widely used as an intestinal model for absorption, transport, and bioavailability studies in recent decades (Fogh & Trempe 1975; Haddad et al. 2023). Confluent Caco2 cells spontaneously start to differentiate from 14 to 21 days and form a polarized cell monolayer with apical and basolateral membranes, a junction complex, and a brush border with microvilli on the apical side typical of human enterocytes (Engle et al. 1998, Hidalgo et al. 1989).

To identify the correct differentiation time to study the effect of E8 on a healthy cell state, Caco2 were treated at 14 and 21 days of differentiation. After exposure to an increasing dosage of E8 (0.05 - 1.0 %), cytotoxicity effects of the composition on Caco2 cells differentiated for 21 days were not observed (Figure 1A, B). Notably, E8 (0.1 and 1.0%) increased the Caco2 viability, probably due to the stimulation of mitochondrial function, since MTT assay indirectly evaluates the activity of mitochondrial succinate dehydrogenase (Figure 1A, B).

Thus, the effect of E8 (0.1 - 1.0 %) on the mRNA relative expressions of the most important proteins involved in the intestinal permeability regulation in Caco2 cells differentiated for 14 or 21 days has been evaluated: 1) zonula occludens-1 (ZO-1), a peripheral membrane adaptor protein that acts as a bridge to connect integral membrane proteins to the actin cytoskeleton and other signalling proteins; 2) occluding and claudins, integral transmembrane proteins; 3) E-cadherin, a major constituent of adherents junctions, involved in the intestinal homeostasis and barrier function (Lee et al. 2018; Schneider et al. 2010). The data showed that E8 treatment for 24 and 48 h increased the gene's relative expression that regulates gut permeability, mainly in Caco2 cells differentiated for 14 days (Figure 1C-G).

Since E8 provided essential amino acids and TCA cycle intermediates involved in mitochondrial regulation, the mRNA expression of two critical mitochondrial genes has also been evaluated: 1) Tfam, which is essential for transcription, replication, and packaging of mtDNA into nucleoids (Stiles et al. 2016); 2) Cyt c (i.e., cytochrome c), a central component of the electron transport chain in mitochondria.

According to the results obtained in the MTT assay, the treatment with the E8 composition stimulates the gene expression of Tfam and Cytoc, confirming the potential benefits of E8 on mitochondrial biogenesis already at basal conditions (Figure 1J, K).

In view of the preliminary results, a 14 days period has been considered as the most valuable differentiation time point in which E8 could ameliorate Caco2 integrity and mitochondrial biogenesis already at basal conditions.

### E8 preserved the intestinal barrier integrity in the in vitro model of gut inflammation

Caco2 cells have also been shown to be able to produce inflammatory markers (e.g., cytokines) in response to specific stimuli such as IL-1β, TNF-α, and LPS (Ponce de León-Rodríguez et al. 2019). This characteristic makes differentiated Caco2 cells a valuable *in vitro* model of gut inflammation (Rodriguez-Ramiro et al. 2013, Hollebeeck et al. 2012, Van De Walle et al. 2008). differentiated Caco2 cells were pre-treated with the composition E8 (0.1 and 1.0%) for 1h, and then the cells were exposed to the inflammatory stimuli [i.e., IL-1β (25 ng/ml), TNF-α (100 ng/ml) and LPS (10 µg/ml)] in presence or absence of the composition E8. Since the first response of Caco2 cells after inflammation is the cytokine production that occurs during 24 h, and only after a sustained incubation with inflammatory mediators the permeability damage occurs (e.g., decreased expression of ZO-1, occludin, and claudins), the evaluation was extended for 24, 48, and 72 h. (Figure 2A, B).

The MTT assay showed increased cell viability after E8 composition treatment in all the experimental conditions, whereas the inflammation did not affect cell health (Figure 2C). CaCo2 cell viability was also evaluated after inflammation and E8 treatments with TOX6 assay (i.e., a measure of total biomass), and no significant cytotoxicity was observed (Figure 2D). Then the E8 composition (0.1 - 1.0 %) efficacy to preserve gut permeability during inflammation at different time was evaluated (Figure 3).

As previously shown, at basal conditions, E8 treatments increased the mRNA relative expression of the primary regulator genes of gut permeability, which was maintained for 72 h (Figure 3A-G). Furthermore, E8 treatment at the higher concentration (1.0%) stimulated the expression of all the genes analysed, even in the presence of inflammation (Figure 3A-G). The pathogenesis of inflammatory bowel diseases is associated with impairing the proteins in gut permeability regulation (Schneider et al., 2010, Bruewer et al., 2006). Notably, in the present experimental conditions, 48 h-inflammation induced the decrease of the gene expression of occludin, claudin 3, claudin 4, and E-cadherin, efficiently prevented by E8 treatment (1.0 %) (Figure 3B and D-F). Furthermore, the E8 treatment at the higher concentration (1.0 %) prevented the damage induced by 48h of inflammation, particularly at the occludin protein levels (Figure 3G, H).

The effect of E8 treatments on the Caco2 cell barrier integrity was therefore evaluated by measuring the trans-epithelial electrical resistance (TEER), which quantifies the movement of ions across the paracellular pathway (Figure 4A).

Caco2 cells were pre-treated with E8 (0.1 - 1.0 %) for 24 h, then the inflammatory stimuli for 24 h was applied. As expected, inflammatory stimuli impaired the gut barrier integrity (-29.3 % vs CTRL) and only the E8 treatment 1.0 % partially preserved cells from the damage (-17 % vs CTRL, and + 12 % vs inflammation) (Figure 4B).

### E8 stimulated the mitochondrial biogenesis in the in vitro model of intestinal inflammation

The effect of E8 treatment (0.1 - 1.0 %) was analysed on mitochondrial biogenesis in the *in vitro* model of gut inflammation. After 48 h, the inflammation impaired the gene expression of PGC1α and COXIV, and the PGC1α protein level of Caco2 cells (Figure 5A-C).

E8 (1.0 %), already active to improve mitochondrial biogenesis at basal conditions, after 48-72 h treatment, increased PGC1α gene and protein expression, as well as COX IV gene expression even in the presence of inflammation (Figure 5A-C).

These results suggest that E8 prevents the mitochondrial damage induced by inflammation.

### E8 attenuated the inflammatory response in the in vitro model of intestinal inflammation

IL8 is a chemokine produced by macrophages and other cell types, including enterocytes, that secreted it to attract and activate neutrophils during the acute phase of inflammation (Hoffmann et al. 2002). II,8 induces chemotaxis of neutrophils and granulocytes, causing them to migrate toward the site of infection and stimulating phagocytosis once they have arrived.

To evaluate the potential anti-inflammatory effect of E8, the IL8 release in the *in vitro* model of gut inflammation was analysed. As expected, the inflammatory mediators stimulated the IL8 release from Caco2 enterocytes after 24 h of exposure, which was maintained for 72 h (Figure 6). The E8 treatment at the lowest concentration (0.1%) reduced the IL8 secretion after 48 h of treatment (-9% vs inflammation, Figure 6). After a sustained treatment, E8 at both dosages (0.1 - 1.0 %) markedly attenuated the inflammatory response, decreasing the IL8 release compared to the inflammation condition to -13% and -9%, respectively (Figure 6).

### The TCA cycle intermediates boosted the healthy benefits of amino acid compositions in the in vitro model of intestinal inflammation

The E8 composition, containing balanced essential and branched-chain amino acids stoichiometric ratios and TCA cycle intermediates (i.e., citric, succinic, and malic acids), has been conceived to counteract the mitochondrial damage and ameliorate the gut barrier function in inflammatory bowel diseases.

To demonstrate that TCA cycle intermediates cooperate with essential amino acids and boost the benefits on intestinal permeability and intestinal mitochondrial biogenesis, we compared the effect of E8 (i.e., essential amino acid composition with TCA cycle intermediates) with an amino acid composition provided to essential amino acid and without TCA cycle intermediates (EAAm, Table 1) on the *in vitro* model of gut inflammation (Figure 7A).

Of note, in the absence of inflammation, the EAAm treatment for 24 and 48 h negatively affected the cell viability; on the contrary, the E8 treatment did not show any cytotoxic effect (Figure 7B). Furthermore, the results show that only E8 prevented the inflammatory-induced damage on the gut barrier, stimulating the expression of the principal regulator genes of gut permeability and mitochondrial biogenesis more efficiently than EAAm (Figure 7C-J).

The effects of a partial composition constituted by the most representative amino acids in the E8 composition (i.e., leucine, lysine, isoleucine, valine, and threonine - "5aa" composition) were also studied in comparison with the same composition added to TCA cycle intermediates at the same ratio provided in E8 ("5aa-acids" composition; Table 1). Differentiated Caco2 cells were treated with the compositions 5aa and 5aa-acids, and E8 (each 1 %) in the presence or absence of inflammatory stimuli for 24 h (Figure 8A).

The results herein provided show that under the basal conditions, the 5aa-acids composition was able to stimulate the mRNA expression of the gut permeability genes more efficiently than the 5aa composition, and even more efficiently the E8 composition (Figure 8B-G). During inflammation, the E8 composition was also able to counteract the damage induced by inflammatory stimuli (Figure 8B-G).

Altogether, the data obtained from comparison with the essential amino acid composition without TCA cycle intermediates (i.e., EAAm) and 5aa-based compositions (i.e., 5aa and 5aa-acids) demonstrated that TCA cycle intermediates represent important boosters in the intestinal epithelium health also allowing to achieve full beneficial effects during inflammation.

Altogether the results herein provided demonstrated that the compositions of the instant application are capable of improving gut permeability and intestinal mitochondrial biogenesis at basal conditions in the *in vitro* model of the intestinal barrier. Notably, the compositions prevented inflammatory damage induced on gut permeability and mitochondrial compartment in the *in vitro* model of gut inflammation. The compositions of the instant application has shown an anti-inflammatory action, reducing the IL8 secretion by enterocytes. The TCA cycle intermediates boosted the effects of essential amino acids on gut permeability and intestinal mitochondrial biogenesis. Overall, the data provided the effect of the compositions in preventing and managing the IMB integrity in patients with intestinal barrier damage or insufficiency.

### References

Bruewer, M., Samarin, S., Nusrat, A. Inflammatory bowel disease and the apical junctional complex. Ann. N Y Acad. Sci. 2006; 1072, 242-252.
Corsetti G, D'Antona G, Ruocco C, et al. Dietary supplementation with essential amino acids boosts the beneficial effects of rosuvastatin on mouse kidney. Amino Acids. 2014; 46:2189-2203.
Engle, M.J.; Goetz, G.S.; Alpers, D.H. Caco-2 cells express a combination of colonocyte and enterocyte phenotypes. J. Cell. Physiol. 1998, 174, 362-369.
Fogh, J., and G. Trempe. 1975. New human tumor cell lines. In Human tumor cells in vitro. ed. J. Fogh, 115-159. New York: Springer.
Haddad, M.J.; Sztupecki, W.; Delayre-Orthez, C.; Rhazi, L.; Barbezier, N.; Depeint, F.; Anton, P.M. Complexification of In Vitro Models of Intestinal Barriers, A True Challenge for a More Accurate Alternative Approach. Int. J. Mol. Sci. 2023, 24, 3595.
Hidalgo IJ, Raub TJ, Borchardt RT. Characterization of the human colon carcinoma cell line (Caco-2) as a model system for intestinal epithelial permeability. Gastroenterology. 1989; 96:736-749.
Hiebl V, Schachner D, Ladurner A, Heiss EH, Stangl H, Dirsch VM. Caco-2 Cells for Measuring Intestinal Cholesterol Transport - Possibilities and Limitations. Biol Proced Online. 2020; 22:7.
Hoffmann E, Dittrich-Breiholz O, Holtmann H, Kracht M. Multiple control of interleukin-8 gene expression. J Leukoc Biol. 2002; 72:847-855.
Hollebeeck S, Winand J, Hérent MF, et al. Anti-inflammatory effects of pomegranate (Punica granatum L.) husk ellagitannins in Caco-2 cells, an in vitro model of human intestine. Food Funct. 2012; 3:875-885.
Lee B, Moon KM, Kim CY. Tight Junction in the Intestinal Epithelium: Its Association with Diseases and Regulation by Phytochemicals. J Immunol Res. 2018; 2018:2645465.
Ponce de León-Rodríguez MDC, Guyot JP, Laurent-Babot C. Intestinal in vitro cell culture models and their potential to study the effect of food components on intestinal inflammation [published correction appears in Crit Rev Food Sci Nutr. 2019;59(13):2166-2168]. Crit Rev Food Sci Nutr. 2019; 59:3648-3666.
Ragni M, Ruocco C, Tedesco L, Carruba MO, Valerio A, Nisoli E. An amino acid-defined diet impairs tumour growth in mice by promoting endoplasmic reticulum stress and mTOR inhibition. Mol Metab. 2022; 60:101478.
Rodríguez-Ramiro I, Ramos S, López-Oliva E, et al. Cocoa polyphenols prevent inflammation in the colon of azoxymethane-treated rats and in TNF-α-stimulated Caco-2 cells. Br J Nutr. 2013; 110:206-215.
Ruocco C, Ragni M, Rossi F, et al. Manipulation of Dietary Amino Acids Prevents and Reverses Obesity in Mice Through Multiple Mechanisms That Modulate Energy Homeostasis. Diabetes. 2020; 69:2324-2339.
Schneider MR, Dahlhoff M, Horst D, et al. A key role for E-cadherin in intestinal homeostasis and Paneth cell maturation. PLoS One. 2010; 5:e14325.
Stiles, A. R., Simon, M. T., Stover, A., Eftekharian, S., Khanlou, N., Wang, H. L., Magaki, S., Lee, H., Partynski, K., Dorrani, N., Chang, R., Martinez-Agosto, J. A., Abdenur, J. E. Mutations in TFAM, encoding mitochondrial transcription factor A, cause neonatal liver failure associated with mtDNA depletion. Molec. Genet. Metab. 2016, 119: 91-99.
Van De Walle J, Romier B, Larondelle Y, Schneider YJ. Influence of deoxynivalenol on NF-kappaB activation and IL-8 secretion in human intestinal Caco-2 cells. Toxicol Lett. 2008; 177:205-214.
Wang W, Liu Q, Wang C, Meng Q, Kaku T, Liu K. Effects of JBP485 on the expression and function of PEPT1 in indomethacin-induced intestinal injury in rats and damage in Caco-2 cells. Peptides. 2011; 32:946-955.

## Claims

1. Composition for use in the prevention and/or in the treatment of an intestinal disease, the composition comprising an active agent, said active agent containing the amino acids leucine, isoleucine, valine, threonine, lysine and citric acid, succinic acid, malic acid or salts thereof.

2. Composition for use according to claim 1, wherein said intestinal disease is selected in the group consisting of irritable intestinal disease (IBS), inflammatory intestinal disease (IBD), wherein the IBD is preferably selected between Crohn's disease and ulcerative colitis.

3. Composition for use according to claim 1 or claim 2, wherein the weight ratio between the sum of citric acid, malic acid, succinic acid and the sum of the branched chain amino acids leucine, isoleucine, valine plus lysine and threonine is comprised between 0.05 and 0.3, preferably between 0.1 and 0.25.

4. Composition for use according to any one of the preceding claims, wherein the weight ratio between the overall amount of citric acid, malic acid, succinic acid and the overall amount of the branched chain amino acids leucine, isoleucine, valine is comprised between 0.1 and 0.4, preferably between 0.15 and 0.35.

5. Composition for use according to any one of the preceding claims, wherein the weight ratio between citric acid and the sum of malic acid and succinic acid is comprised between 1.0 and 4.0, preferably between 1.5 and 2.5.

6. Composition for use according to any one of the preceding claims, wherein the citric acid:malic acid:succinic acid weight ratio is comprised between 10:1:1 and 2:1.5:1.5, preferably between 7:1:1 and 1.5:1:1, more preferably between 5:1:1 and 3:1:1.

7. Composition for use according to any one of the preceding claims, wherein said active agent further comprises at least one amino acid selected in the group consisting of histidine, phenylalanine, methionine, tryptophan, tyrosine, cysteine.

8. Composition for use according to any one of the preceding claims, wherein said active agent further comprises histidine, phenylalanine, methionine, tryptophan, cysteine.

9. Composition for use according to any one of the preceding claims, wherein the ratio between the overall weight amount of citric acid, malic acid, succinic acid and the overall weight amount of methionine, phenylalanine, histidine and tryptophan is higher than 1.35.

10. Composition for use according to any one of the preceding claims, wherein the ratio between the overall weight amount of the three acids citric acid, succinic acid, malic acid and the overall weight amount of lysine and threonine is comprised between 0.10 and 0.70, preferably between 0.15 and 0.55.

11. Composition for use according to any one of the preceding claims, wherein the amino acids isoleucine, leucine and valine are present in an amount between 35% and 65% by weight, preferably between 42% and 58% by weight with respect to the composition weight.

12. Composition for use according to any one of the preceding claims, wherein the weight or molar amount of citric acid is higher than the overall weight or molar amount of both malic acid and succinic acid.

13. Composition for use according to any one of the preceding claims, wherein the weight ratio between leucine and citric acid is comprised between 1 and 5, preferably between 2.50 and 3.90.

14. Composition for use according to any one of the preceding claims, wherein said composition is free of arginine.

15. Composition for use according to any one of the preceding claims, wherein said composition is free of serine, proline, alanine.
